# EUROPEAN PATENT SPECIFICATION

(11) **EP 1 787 731 B1**
(45) Date of publication and mention of the grant of the patent: **20.01.2016**
(21) Application number: 06124017.2
(22) Date of filing: 14.11.2006
(51) Int. Cl.: B08B 3/04, A61L 2/04

(54) **Washing apparatus,in particular for heat-disinfection**
Waschvorrichtung, insbesondere zur thermischen Desinfektion
Appareil de lavage, en particulier pour désinfection thermique

(30) Priority: 16.11.2005 IT UD20050194; 21.12.2005 IT UD20050216; 25.10.2006 IT UD20060228
(43) Date of publication of application: 23.05.2007
(60) Divisional application: 15199472.0
(73) Proprietor: STEELCO SPA, 31039 Riese Pio X (IT)
(72) Inventor: Zardini, Fabio, 31033 Castelfranco Veneto (TV) (IT)
(74) Representative: Petraz, Gilberto Luigi

(56) References cited:
- FR-A- 2 098 749
- FR-A- 2 803 528
- GB-A- 2 321 596

## Description

### FIELD OF THE INVENTION

The present invention concerns a washing apparatus, in particular for the heat-disinfection of objects, for example medical instruments for hospital wards, operating rooms, laboratories and the pharmaceutical industry, comprising a plurality or battery of washer machines aligned in a determinate disposition, and a movement device for moving the relative object-bearing containers in front of the washer machines and inside them.

### BACKGROUND OF THE INVENTION

The document GB-2 321 596 A, which is considered as the closest state of the art discloses a washing apparatus in accordance with the preamble of claim 1.

Usually, hospital structures have a plant for treating objects such as for example instruments used in the operating rooms and therefore potentially infected and not sterile, before they can be used again. This treatment usually consists of a cold-wash or pre-washing step, of a washing step, heat-disinfection and sterilization.

In particular, the plant is normally divided into several sectors, isolated from each other for reasons of hygiene and known respectively as the "dirty" sector, or reception, the "clean" sector, and the "sterile" sector.

In the first sector, the dirty objects that are to be subjected to the various treatments arrive. The treatments carried out are generally: pre-washing with cold water only, possible washing in an ultrasound bath, washing in hot water and possibly detergents, the necessary rinses, heat-disinfection and final drying. In particular, heat-disinfection, which is a particular type of washing, is done with hot water, usually at a temperature comprised between about 90°C and about 93°C.

After they have been heat-disinfected, the objects pass to the second "clean" sector where they are possibly packaged and from here they are fed to sterilization machines, generally consisting of autoclaves, which sterilize them.

The objects, thus sterilized, pass to the following sterile sector where they are stored or sent again for use in the operating room.

In the treatment plant, different washing apparatuses are used to carry out the various treatments of pre-wash, washing, heat-disinfection and sterilization. The pre-wash, washing and heat-disinfection treatments can be done by means of the same apparatus, or by means of separate apparatuses, according to requirements.

Known washing apparatuses for the treatment of objects comprise a plurality of washer machines, disposed aligned in batteries, or in parallel, along a determinate path, so that the entrance to each of them is parallel to the entrance of the other adjacent washer machines. In this way, the set or row of individual front walls of the washer machines defines overall a single front wall, substantially continuous.

Generally, each washer machine has an internal chamber, in which the washing cycle takes place, and which is accessed through an aperture made at a determinate height and able to be selectively closed by a shutter-type door.

Known washing apparatuses also comprise a movement device, both to automatically move the object-bearing containers parallel to the front wall of the washer machines, and also to feed them inside the washer machines.

In particular, a movement device is known which comprises a trolley mounted on a mobile frame which is supported slidingly by a rail, mounted above the washer machines. In this known device, the mobile frame also rests in abutment on a lower track, disposed in proximity with the floor and adjacent to the front wall defined by the washer machine.

The movement of the trolley along the battery of washer machines is obtained by means of a transmission belt driven by an electric motor.

The movement device has the disadvantage that it is complex and not very practical, especially because the mobile frame is supported by the fixed rail disposed above the washer machines and is mounted cantilevered and in pendulum fashion.

In this way, the known movement device does not guarantee the correct positioning, that is, the centering, of the trolley with respect to the entrance aperture to the washing chamber of the selected machine, with the obvious risk of damage to the objects contained in the containers during the steps when the containers are introduced and removed.

Another disadvantage of the known movement device is that they are particularly difficult to clean and maintain. This is particularly serious in a hospital environment.

Another disadvantage of the known movement device is that it does not allow to open the door of the washer machine upwards, due to the upper interference with the mobile frame. This is a disadvantage if it is necessary to carry out maintenance on the bottom of the washer machine and with the door open, which would impede the operation.

Purpose of the present invention is to achieve a washing apparatus having a movement device that allows, simply and economically, the correct and precise positioning of the containers with respect to the selected washer machine, and so that this can take place irrespective of the type of washer machine used.

The Applicant has devised, tested and embodied the present invention to overcome the shortcomings of the state of the art and to obtain these and other purposes and advantages.

### SUMMARY OF THE INVENTION

The present invention is set forth and characterized in the independent claim, while the dependent claims describe other characteristics of the invention or variants to the main inventive idea.

In accordance with the above purpose, a washing apparatus, in particular for heat-disinfection, comprises a plurality of washer machines disposed aligned along an axis of alignment, and a movement device able to move at least an object-bearing container parallel to said axis of alignment.

In particular, the movement device comprises a trolley able to transport the object-bearing container and provided with sliding means.

The sliding means is guided by a guide element, which extends parallel to said axis of alignment.

The movement device also comprises movement means to move the trolley parallel to the guide element.

According to a characteristic feature of the invention, the washing apparatus comprises a frame associated, at least partly, with said movement means, and disposed along said axis of alignment so as to define a plurality of housing zones, in which the washer machines are positioned.

In this way the washer machines, although they are totally unconstrained and autonomous with respect to the frame, are positioned precisely with respect to it, so that a determinate positioning of the trolley with respect to the frame also defines a correct positioning of the trolley with respect to the washer machines.

The solution according to the present invention thus allows an efficient positioning of the trolley with respect to an entrance aperture of each of the washer machines even if the trolley itself is not constrained to the washer machines. With this solution, moreover, the advantageous reference of the frame prevents dangerous oscillations and twisting of the trolley.

The housing zones defined by the frame according to the present invention can be of any size, depending on the type of washer machine to be used, and can therefore be chosen as desired, according to the type of treatment to be done.

Advantageously, the sliding means slides on support means autonomous with respect to the washer machines.

The present invention, by rendering both the frame and also the support means of the trolley independent from the washer machines, prevents any kind of interference between the trolley and the washer machines in correspondence with the entrance aperture of the latter, thus improving operating safety.

Advantageously, the solution of making the weight of the trolley lie completely on the sliding means, and hence on the support means which is autonomous with respect to the washer machines and disposed under the loading plane of the washer machines, completely frees the space above the washer machines. Therefore, the apparatus can be installed even in premises with relatively low ceilings.

Advantageously, the guide element has the function of constraining the trolley only to the translation motion parallel to the axis of alignment, to prevent any transverse movement thereof with respect to the axis. This is essential to correctly position the container with respect to the internal chambers of the washer machines, and to feed it inside them, without damaging the objects contained therein.

### BRIEF DESCRIPTION OF THE DRAWINGS

These and other characteristics of the present invention will become apparent from the following description of a preferential form of embodiment, given as a non-restrictive example with reference to the attached drawings wherein:
- fig. 1 is a plane schematic view of a plant for the complete treatment of pre-wash, washing, heat-disinfection and sterilization, comprising a washing apparatus according to the present invention;
- fig. 2 is a schematic plane view of the washing apparatus in fig. 1;
- fig. 3 is an enlarged lateral view of a part of the apparatus in fig. 2;
- fig. 4 is a section view from IV to IV of fig. 3;
- fig. 5 is an enlarged detail of fig. 3;
- fig. 6 is a perspective view of a washing apparatus according to the present invention, associated with a pre-wash apparatus;
- fig. 7 is a plane view of a washing apparatus according to the present invention, associated with a pre-wash apparatus;
- fig. 8 is a perspective view of a form of embodiment of a movement device of the washing apparatus according to the present invention;
- fig. 9 is a schematic lateral view of the movement device in fig. 7;
- fig. 10 is an enlarged detail of fig. 8; and
- fig. 11 is a perspective view of another form of embodiment of a movement device of the washing apparatus according to the present invention.

### DETAILED DESCRIPTION OF A PREFERENTIAL FORM OF EMBODIMENT

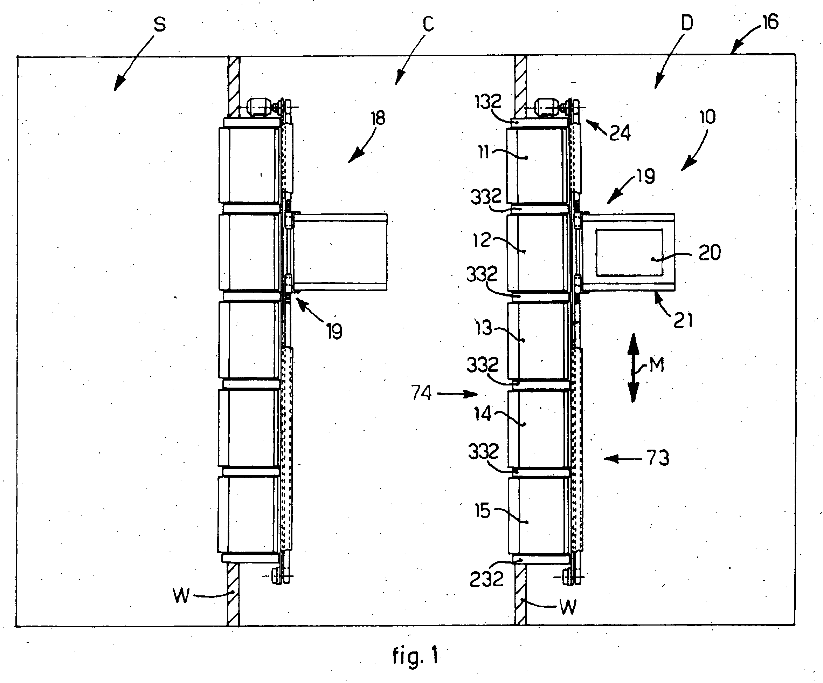

With reference to fig. 1, a washing apparatus 10, in particular for heat-disinfection, according to the present invention is used for example in a plant 16 in which the complete treatment of pre-wash, washing, heat-disinfection and sterilization of instruments arriving from hospital wards, operating rooms, laboratories and the pharmaceutical industry is performed.

The plant 16 is therefore made with various sectors divided, for example by walls W, in particular a dirty sector D, where the dirty instruments are received, a clean sector C and a sterile sector S.

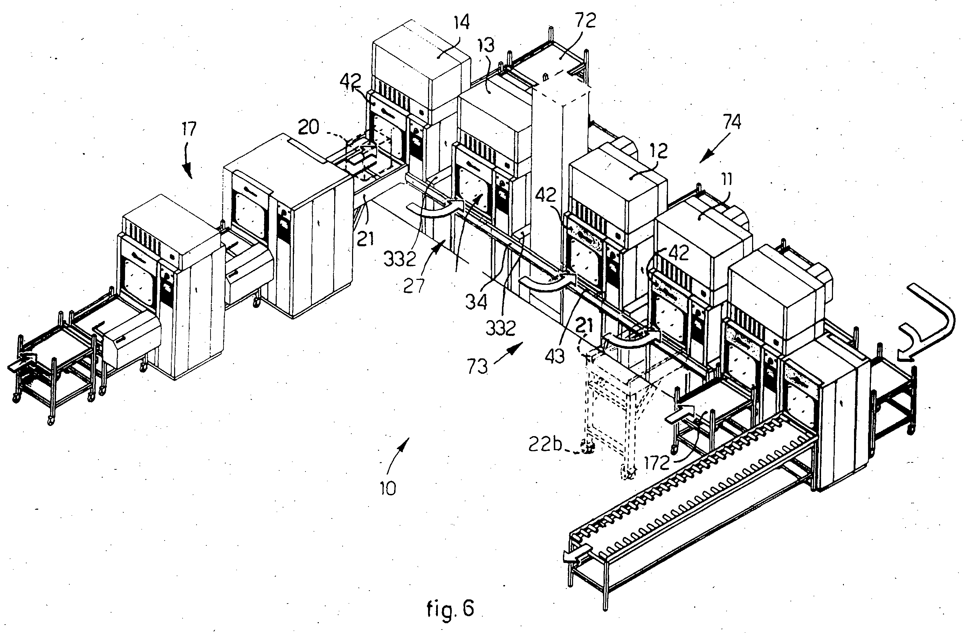

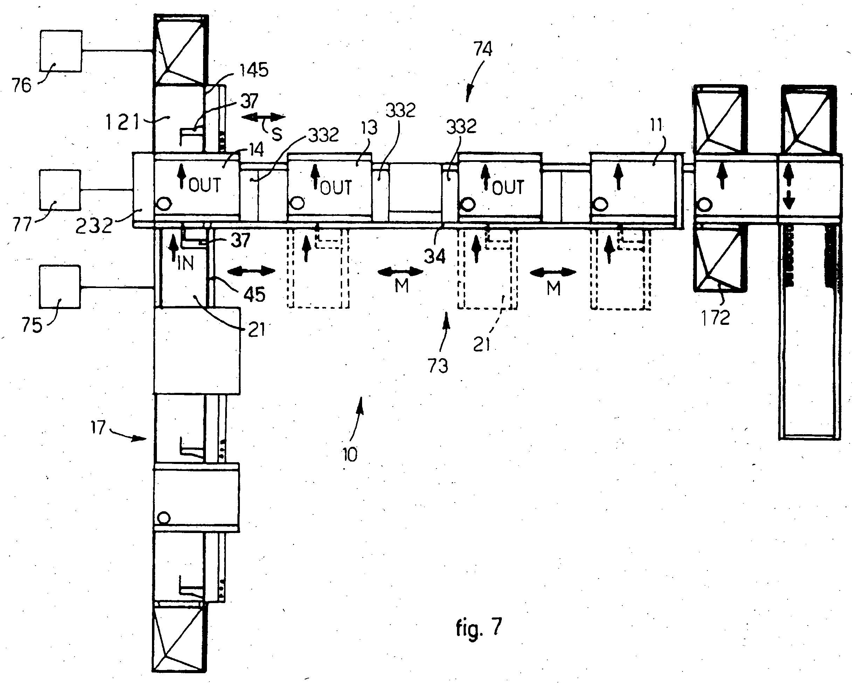

Usually the plant 16 comprises, in the dirty sector D and upstream of the apparatus 10 where heat-disinfection is performed, a first battery 17 of washer machines for a cold-water pre-wash, washing possibly with chemical detergents and/or washing in an ultrasound bath, not shown in fig. 1 and shown for convenience in figs. 6 and 7.

Moreover, downstream of the washing apparatus 10, the plant 16 generally comprises a second battery 18 of sterilizing machines for the instruments already subjected to heat-disinfection.

Therefore, the dirty sector D is upstream of the washing apparatus 10, the washing apparatus 10 and the second battery 18 delimit the clean sector C, while the sterile sector S is downstream of the second battery 18.

Here and hereafter, the term washing apparatus shall comprise at least a cold-water pre-wash machine, washing possibly with detergents and/or an ultrasound wash, or at least a heat-disinfection machine or at least a sterilizing machine, according to requirements.

In this case, the washing apparatus 10 is described in its application to the washing and heat-disinfection treatment.

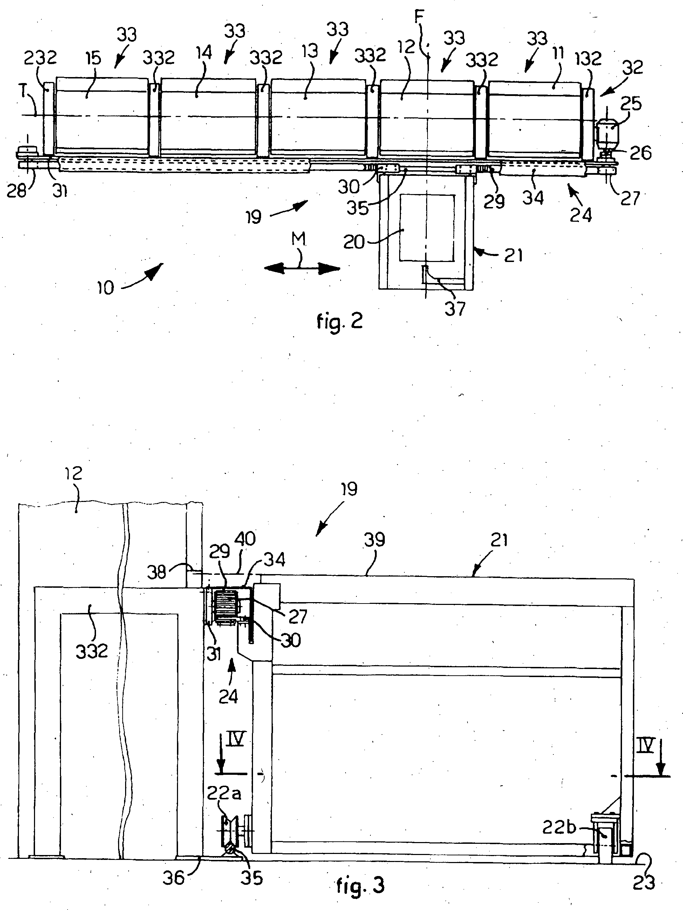

The washing apparatus 10 comprises a plurality or battery of washer machines, in this case washing and heat-disinfection machines, 11, 12, 13, 14, 15, which are positioned aligned along an axis of alignment T (fig. 2).

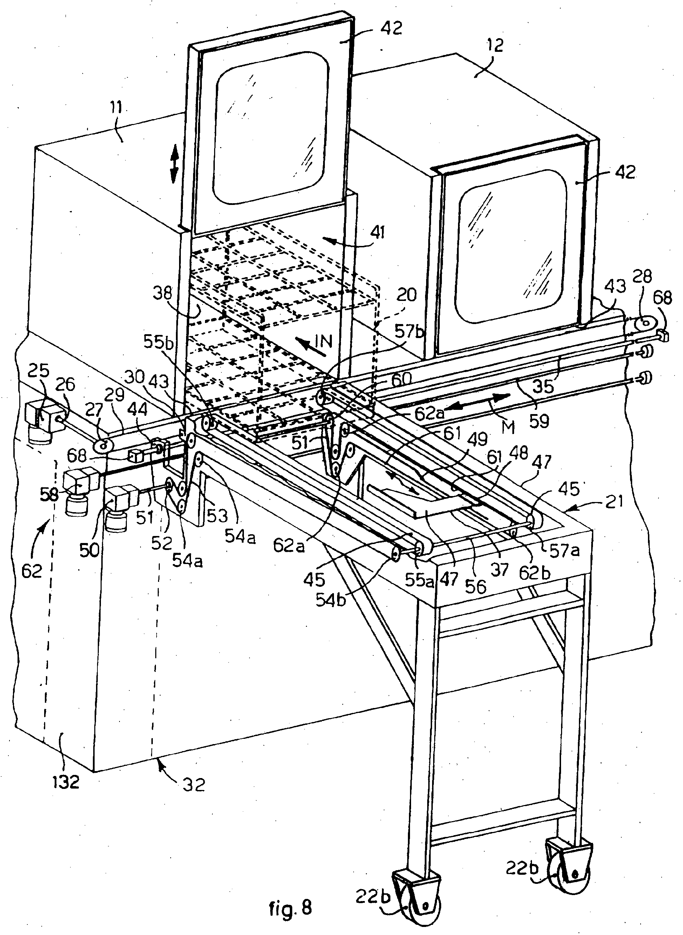

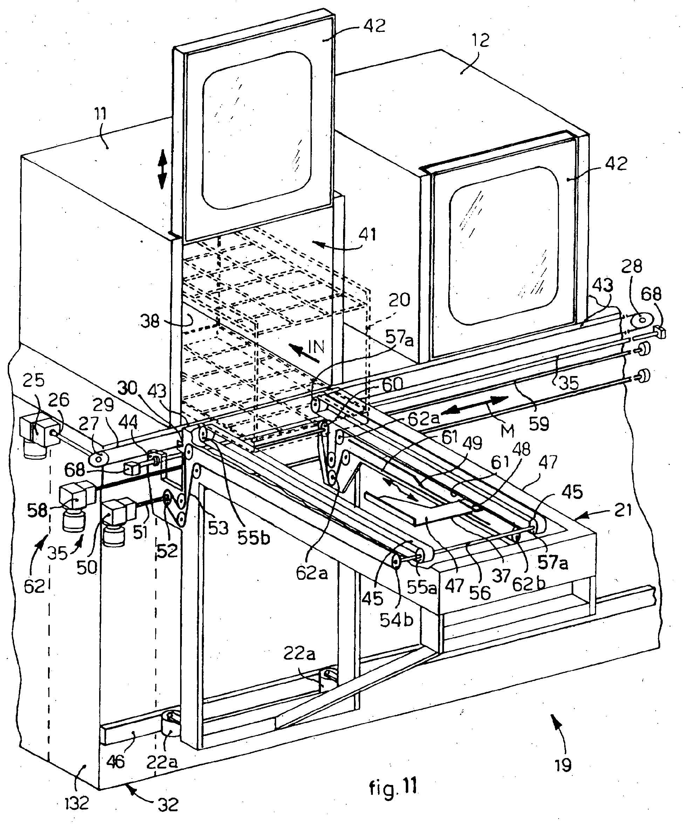

For example in figs. 1 and 2 five washer machines 11, 12, 13, 14, 15 are indicated, whereas in figs. 6 and 7 four washer machines 11, 12, 13, 14 are indicated and in figs. 8 and 11 two washer machines 11, 12 are shown.

The battery of washer machines 11, 12, 13, 14, 15 thus defines an entrance side 73, from which the dirty instruments to be washed are loaded, and an exit side 74, from which the washed instruments emerge. As we said, the entrance side 73 and the exit side 74 are preferably separate and distinct, as shown in figs. 1, 6 and 7.

It is clear that, according to necessity, the axis of alignment T could develop along a curve or a straight line, without substantially changing the application of the present invention.

Each washer machine 11, 12, 13, 14, 15 is provided with an aperture 38 (fig. 3) disposed at a predeterminate height, which functions as an entrance to an internal chamber 41 where heat-disinfection takes place, and has a lower end 43.

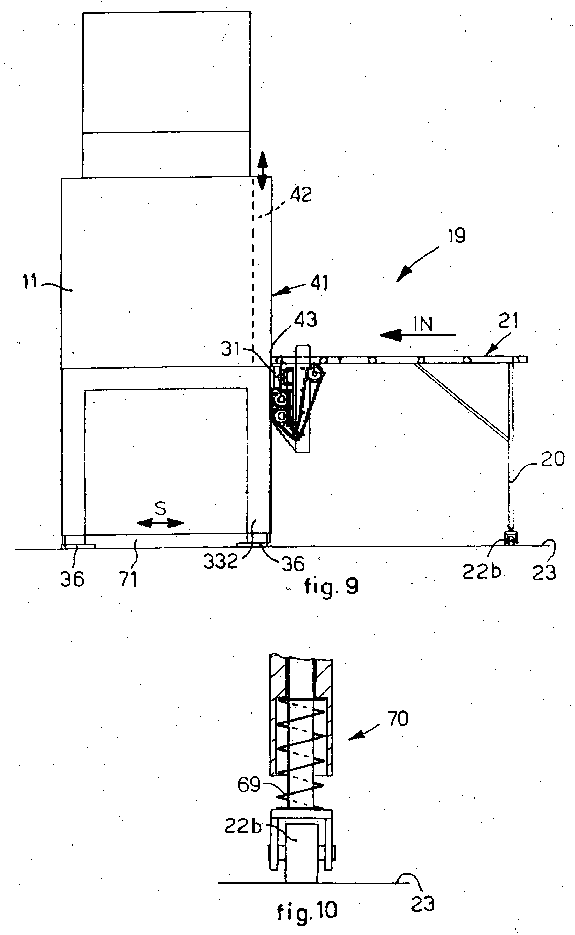

The aperture 38 is selectively closed by means of a door 42 of the shutter type which, when it is closed, abuts on the lower end 43 (figs. 6 and 9).

The apparatus 10 also comprises a movement device 19 (figs. 1, 2, 3, 8 and 9) for moving at least an instrument-bearing container 20 parallel to said axis of alignment T and in front of the washer machines 11, 12, 13, 14, 15, as indicated by the arrow M in figs. 1 and 2, and for feeding it to a selected washer machine 11, 12, 13, 14, 15.

It is clear that the movement device 19 can be used without distinction both on the washing apparatus 10 intended as a heat-disinfection battery, and on the first pre-wash battery, and also on the second sterilization battery 18, as can be seen, to give a non-restrictive example in fig. 1.

The present invention, as will be shown in the following part of the description, allows to de-constrain the washer machines 11, 12, 13, 14, 15 from the movement device 19, also allowing to vary the type and/or sizes of the washer machines 11, 12, 13, 14, 15.

The movement device 19 comprises a trolley 21 to transport the container 20 (figs. 1, 2, 3, 4 and 5). The trolley 21 is able to translate along the axis of alignment T, commanded and controlled by a suitable command and control unit, as will be shown in the following part of the description.

The trolley 21 comprises an upper plane 39, where the container 20 is able to rest, it is at a determinate height, according to the height of the entrance aperture 38 of the washer machine 11, 12, 13, 14, 15 and possibly adjustable, according to necessity and to the type of washer machine 11, 12, 13, 14, 15 used, in a known manner.

The upper plane 39 of the trolley 21 defines, in correspondence with the aperture 38 or slightly below it, a loading plane 40 along which the container 20 is loaded into the selected washer machine 11, 12, 13, 14, 15 (fig. 3). Therefore, advantageously, the movement device 19 is in correspondence with the aperture 38 or below it.

The loading plane 40 is substantially on the same level as, or slightly below, the lower end 43 of the door 42. Therefore, the trolley 21 is always moved on the level of the lower end 43 or below it.

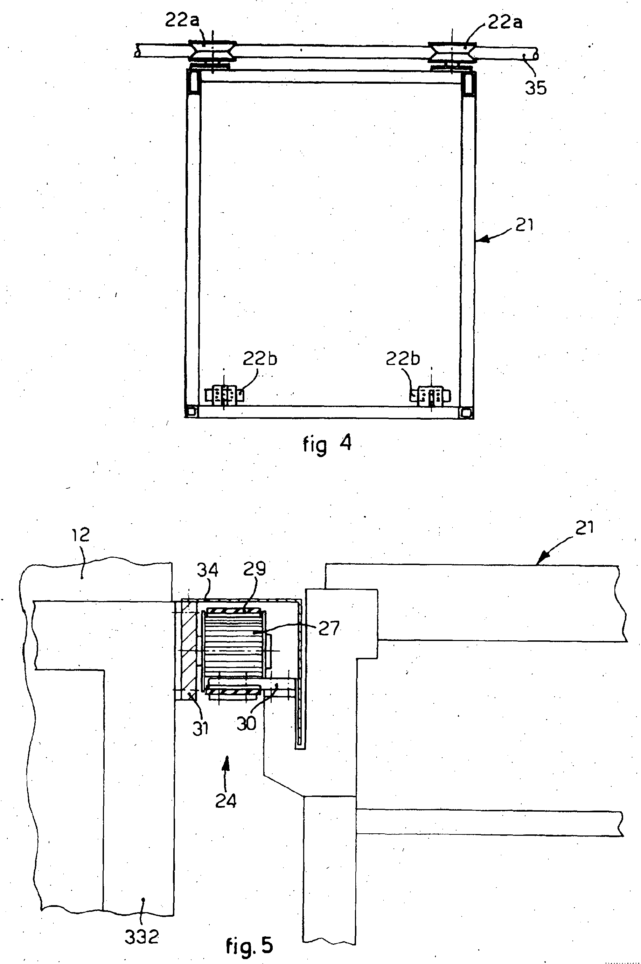

According to a first form of embodiment of the present invention, the trolley 21 is provided with vertical legs 70 at the ends of which wheels 22a, 22b are disposed (figs. 3 and 4), which are able to slide and cooperate, directly or indirectly, with a support plane 23. In fig. 4 the trolley 21 is shown, to give a non-restrictive example, with four wheels, respectively two indicated by 22a and two indicated by 22b.

All the weight of the trolley 21 therefore lies on the wheels 22a, 22b and, from here, on the support plane 23.

The support plane 23 is autonomous with respect to the washer machines 11, 12, 13, 14, 15; in this case it is a horizontal floor and is disposed below the loading plane 40 of the washer machines 11, 12, 13, 14, 15, freeing the part above the loading plane 40 (fig. 3).

The trolley 21 is moved parallel to the axis of alignment T by movement means 24. According to a non-restrictive form of embodiment of the invention, the movement means 24 comprises a motor 25 (fig. 2) able to drive, by means of its drive shaft 26, a drive pulley 27. The drive pulley 27 is associated with a corresponding driven pulley 28 and drives a transmission belt 29 which extends and operates parallel to the axis of alignment T (figs. 2 and 3).

The trolley 21 is provided with a connection portion, or blade, 30 (figs. 3 and 5) by means of which it is attached and made solid to said transmission belt 29. The transmission belt 29 thus has the function of drawing the trolley 21 in movement parallel to the axis of alignment T and does not function as a support for the trolley 21.

The movement means 24 is supported, at least partly, by a frame 32 (fig. 2) which is totally unconstrained and autonomous from the washer machines 11, 12, 13, 14, 15 and is disposed along the axis of alignment T. The frame 32 is made in such a manner as to define a plurality of housing zones 33, in which the washer machines 11, 12, 13, 14, 15 are able to be selectively positioned and/or removed, according to necessity. The machines 11, 12, 13, 14, 15 are therefore unconstrained and autonomous from the frame 32 and the movement device 19.

The frame 32 comprises a first pillar 132 disposed at a first lateral end of the battery of washer machines 11, 12, 13, 14, 15 and which supports the motor 25, and a second pillar 232 disposed at a second lateral end of the battery of washer machines 11, 12, 13, 14, 15.

The first and second pillar 132, 232 support a horizontal bar 31, represented for convenience only in figs. 2, 3, 5 and 9, which extends parallel to the axis of alignment T. The bar 31 supports the drive pulley 27 and the idle pulley 28.

The frame 32 is also provided with a plurality of intermediate pillars 332, disposed distanced from each other, according to the sizes of the washer machines 11, 12, 13, 14, 15, and interspersed with the latter, so as to define the housing zones 33.

The pillars 132, 232, 332 are conformed as trestles or an upside-down U, and are attached to the support plane 23 by means of plates 36 (figs. 3 and 9).

The advantages of the embodiment of the frame 32 as described are the great adaptability of size and the modularity with which the housing zones 33 can be made, according to the type of washer machines 11, 12, 13, 14, 15.

The movement means 24 is covered and protected by a suitable guard 34, mounted on the bar 31 (figs. 2, 3, 5, 6 and 7).

According to a variant of said movement means 24, not shown, there is nothing to prevent the motor 25 from being mounted directly on the trolley 21, and being operatively connected to the wheels 22a, 22b, making them motorized and able to move the trolley 21 autonomously.

However the movement of the trolley 21 is achieved according to this first form of embodiment, the wheels 22a, 22b of the trolley 21 are guided, directly or indirectly, by a longitudinal guide 35 which extends parallel to the axis of alignment T (figs. 2, 3 and 4).

The guide 35 can be disposed in direct or indirect contact with the support plane 23 and can be coupled directly or not with the wheels 22a, 22b. According to a simplified form of embodiment, the guide 35 is made fixed to the plates 36 of the frame 32 (fig. 3).

To give a non-restrictive example for the purposes of the present invention, fig. 3 shows the trolley 21 having a wheel 22a sliding directly on the guide 35, and another wheel 22b sliding resting directly on the support plane 23.

Again in fig. 3, the wheel 22b is shaped so as to have a circumferential groove inserted on the guide 35. Alternatively, the guide 35 itself could be provided, with equivalent result, with a longitudinal seating in which the wheel 22a can slide.

According to a variant not shown in the drawings, the guide 35 may also not be coupled directly with the wheels 22a, 22b, cooperating, instead, with a different portion of the trolley 21 and disposed, for example, in correspondence with said transmission belt 29. In this case, however, in order to discharge in any case the weight of the trolley 21 only onto the support plane 23, the trolley 21 is made with at least three wheels 22a, 22b, for example one wheel 22a and two wheels 22b or vice versa.

According to another variant not shown in the drawings, the guide 35 can consist of magnetic tracks, of a known type, disposed on the support plane 23.

There is no limitation, for the purposes of the invention, as to where and how the guide 35 is made, or how it cooperates with the wheels 22a, 22b of the trolley 21, or with the trolley 21 itself. In fact, according to the first form of embodiment of the present invention, the function of the guide 35 is to constrain the trolley 21 to move only along the axis of alignment T, so as to prevent any transverse movement thereof with respect to the axis of alignment T. Moreover, the guide 35 is disposed at a predeterminate reference distance from the frame 32, so that the containers 20 are loaded into the washer machines 11, 12, 13, 14, 15 easily and safely.

The variant in which the guide 35 is not coupled with the wheels 22a, 22b and supports at least partly the weight of the trolley 21 will now be described, according to a second form of embodiment.

In this case, the guide 35 can be disposed in correspondence with said transmission belt 29 and therefore can be supported by the bar 31. In this case, the guide 35 cooperates with a suitable portion 44 of the trolley 21 and is not directly coupled with the wheels 22a, 22b. According to an advantageous embodiment, in this case the guide 35 is provided, at its ends, with catches 68 (figs. 8 and 11), able to prevent the trolley 21 from becoming detached from the guide 35.

As shown in figs. 6, 8, 9 and 11, the trolley 21 can have only two wheels 22a or only two wheels 22b. In particular, in figs. 8 and 9, the trolley 21 is shown provided with only two wheels 22b which slide directly in contact with the support plane 23. In fig. 11, on the contrary, the trolley 21 is shown provided with only two wheels 22a, with a vertical axis of rotation, which slide on a rail 46 which is disposed longitudinal and parallel to the axis of alignment T and is fixed to the lower portion of the frame 32, in proximity with the support plane 23.

In the variants shown in figs. 8, 9 and 11, the guide 35 also functions as a support for the weight of the trolley 21, allowing it in any case to slide. In particular, in the variant shown in figs. 8 and 9, the weight of the trolley 21 is discharged both onto the support plane 23 and also onto the guide 35, and from here onto the bar 31 and the frame 32. On the contrary, in the variant shown in fig. 11, the weight of the trolley 21 is discharged both onto the rail 46, and therefore onto the frame 32, and also onto the guide 35, and from here onto the bar 31 and the frame 32.

According to another variant not shown in the drawings, the guide 35 can consist of two parts, of which one cooperates with the wheels 22a, 22b, acting as a guide proper on the level of the support plane 23, and the other supporting the weight of the trolley 21, substantially at the height of the bar 21.

In order to load the container 20, the movement device 19 also comprises a conveyor belt 45, or alternatively a roller, disposed on the trolley 21 (figs. 8 and 11). The belt 45 feeds the container 20 towards the internal chamber 41, and optionally facilitates the exit of the container 20 from the internal chamber 41. The container 20 is moved in a direction indicated by the arrow IN (figs. 7, 8 and 11) parallel to an axis of feed F (fig. 2) which in turn is perpendicular to the axis of alignment T.

Moreover, the trolley 21 is also provided with an arm 37 which is mounted movably on the upper plane 39 of the trolley 21 (figs. 2, 7, 8 and 11). The arm 37 is disposed cantilevered along one side 47 of the trolley 21, advantageously along a side parallel to the direction indicated by the arrow IN.

The arm 37 is driven, alternately, to thrust the container 20 into the internal chamber of one of the washer machines 11, 12, 13, 14, 15, but also to remove it therefrom, along the axis of feed F (fig. 2).

The arm 37 is made to advance or retreat according to the position of the container 20 on the trolley 21, and also to rise or lower with respect to the loading plane 40, so as to selectively cooperate with the container 20.

In particular, the arm 37 is provided with a thrust portion and optionally an attachment portion, for example of the oscillating type, which are able to engage and disengage selectively from the container 20.

When the container 20 is completely disposed on the upper plane 39 of the trolley 21, the arm 37 is below the upper plane 39, so as not to impede the movement of the container 20 from and towards the internal chamber 41. The container 20 frees a corresponding space in which the arm 37 can rise when it is moved by the conveyor belt 45 towards the internal chamber 41, no longer completely occupying the upper plane 39.

Automatically, the arm 37 starts to move in the direction indicated by the arrow IN, to thrust the container 20. As it gradually moves in the direction indicated by the arrow IN, the arm 37 is lifted, substantially to the height of the base of the container 20, to cooperate with it, thrusting it completely into the internal chamber 41.

So that the arm 37 can rise and be lowered, it comprises an oscillating portion 47, hinged to a supporting portion 48 of the arm 37 (figs. 8 and 11).

The arm 37 slides along a suitable guide 49, which is made by means of an eyelet or groove, or by means of two offset parallel surfaces connected by an inclined surface, so as to define at least a sliding segment which has a change of slope, on which a sliding portion of the arm 37 slides and substantially operates as a cam.

The arm 37 moves along the guide 49 and its oscillating portion 47 changes its inclination and is lifted in order to thrust the container 20 when the arm 37 moves towards the internal chamber 41 or is lowered in the opposite direction.

According to one form of embodiment of the present invention, given as a non-restrictive example, we shall now describe how the conveyor belt 45 and the arm 37 are moved.

The conveyor belt 45 derives its movement from a motor 50, attached to the first pillar 132, which is able to deliver a torque that is transmitted to a rotation bar 51, to give a non-restrictive example, of the type with a hexagonal section, and disposed parallel to the guide 35 (figs. 8 and 11).

The rotation bar 51 is then made to rotate and transmits rotation directly to a drive pulley 52, slidingly coupled with the rotation bar 51. The drive pulley 52, by means of a transmission chain 53, moves five pulleys 54a, 54b, of which four pulleys are idle, or diversion pulleys 54a, and a transmission pulley 54b. The transmission pulley 54b transmits the rotation by means of a shaft 54c to a driven pulley 55a which, in cooperation with a relative idle pulley 55b, moves the conveyor belt 45, in this case left. Moreover, the driven pulley 55a, by means of a rotation bar 56, makes another driven pulley 57a rotate, which in cooperation with the relative idle pulley 57b drives the conveyor belt 45, in this case right.

In the same way, the arm 37 derives its movement from a motor 58, also supported by the first pillar 132, which delivers a torque that is transmitted to a rotation bar 59, for example also of the type with a hexagonal section, and disposed parallel to the guide 35.

The rotation bar 59 is made to rotate and, in turn, transmits rotation to a drive pulley 60, slidingly coupled with the rotation bar 57. The drive pulley 60, in cooperation with corresponding four idle pulleys 62a and a driven pulley 62b, moves a transmission chain 61, which is attached to the support portion 48 of the arm 37, drawing the latter into movement along the guide 49. By inverting the rotation of the rotation bar 51 and the rotation bar 59, the direction of movement respectively of the conveyor belt 45 and the arm 37 is also inverted.

According to an advantageous embodiment of the present invention, the motors 25, 50 and 58 are disposed and compacted, that is, integrated, in a single drive unit 62, disposed separate, that is, mounted off-board, with respect to the trolley 21, for example disposed at the side of the washer machine at one end of the battery of washer machines 11, 12, 13, 14, 15 and supported by the first pillar 132 of the frame 32.

Advantageously therefore, since it is completely outside the trolley 21, the drive unit 62 is independent and stationary with respect to the movement of the trolley 21, and easily accessible for cleaning and maintenance.

Referring again to the plant 16 shown in fig. 1 and partly in figs. 6 and 7, the first battery 17 consists for example of a series of pre-wash machines 63, 64, disposed as a "tunnel". Each pre-wash machine 63 is fed by a roller 65 and/or a mobile arm 66. The first battery 17 is served, upstream, by a manual trolley 67. The container 20 passes from the exit of the machine 64 to the trolley 21 for example by means of a roller or conveyor belt. Alternatively, on one side the trolley is directly adjacent to the machine 64, so that the container 20 can be transferred, whereas on an opposite side it is in direct contact with the lower end 43 of the entrance aperture 38 of the washer machine 11.

Between the pre-wash and the wash and heat-disinfection an accumulation zone, or storage zone, for the containers 20 is advantageously provided.

If the washer machine 11 is not accessible for washing, for example because it is occupied by another container to be washed, or because it is not functioning, the trolley 21 is able to translate so as to position itself in correspondence with a washer machine 11, 12, 13, 14, 15 which is free and available for washing.

The clean instruments are discharged on the exit side 74 of the washer machines 11, 12, 13, 14, 15 using a corresponding movement device 19, or manual trolleys 72, according to necessity.

If the movement device 19 is used on the exit side 74, it is also provided with a trolley 121, which operates and is moved like the trolley 21, selectively translating along the exit of the washer machines 11, 12, 13, 14, 15 in a direction S parallel to the axis of alignment T (fig. 3).

The containers 20 are moved out from the internal chamber 41 in an exit direction OUT, perpendicular to direction S, by means of a conveyor belt 145 and/or an arm 137 mounted on the trolley 121, and from here are moved to a storage zone or sent for sterilization.

However, in particular cases, for example in emergencies or when parts of the washing apparatus 10 are malfunctioning, the clean instruments or containers can be discharged on the entrance side 73, that is, frontally, for example by using a manual trolley 172, if the trolley 21 is occupied, or using a return roller 65.

In any case, advantageously, the container 20 is loaded and unloaded without using mobile parts or parts active in the washer machines 11, 12, 13, 14, 15, thus preventing the premature wear thereof.

The movement of the trolley 21 and the container 20 on the entrance side 73 is commanded and controlled by an autonomous control unit 75 (fig. 7), while the movement of the trolley 121 and the container 20 on the exit side 74 is commanded and controlled by a different control unit 76, autonomous from the control unit 75.

Furthermore, the washer machines 11, 12, 13, 14, 15 and the washing and heat-disinfection cycle are commanded and controlled by another control unit 77, in turn separate and autonomous from the control units 75 and 76.

The passage of the container 20 from the first battery 16 to the trolley 21 is managed by means of the control unit 76, which receives verification signals from suitable mechanical pressure sensors.

In the same way, a sensor disposed on the trolley 21 signals to the control unit 75 that the container 20 has been loaded onto the trolley 21, to allow the movement of the trolley 21 along the axis of alignment T. When a washer machine 11, 12, 13, 14, 15 is free, it sends a signal to the control unit 77, which forwards it to the control unit 75, so that the trolley 21 is driven by the motor 25 and is positioned exactly in front of the free washer machine.

A sensor detects the exact positioning and centering of the trolley 21 in front of the door 42 and sends a signal to the control unit 77, which commands the door 42 to open. The opening of the door 42 is signalled to the control unit 75, which drives the motors 50 and 58 to move the conveyor belt 45 and the arm 37. The latter complete the loading of the container 20 in the internal chamber 41. A corresponding sensor detects that the container 20 has been fed into the internal chamber 41 and signals this to the control unit 77. The latter commands the door 42 to close, selects a suitable washing and heat-disinfection cycle, for example according to data read from a suitable recognition code present on the container 20, and commands the washing and heat-disinfection cycle to begin.

On the exit side 74 corresponding sensors are disposed which interface with the control units 76 and 77, to unload the containers 20 correctly and automatically once the selected washing cycle is complete.

The use of control units 75, 76, 77 physically separate but interfaced by sensors allows the partial use of parts of the washing apparatus 10 when other parts are not active. For example, it allows the manual loading of the containers 20 and at the same time the automatic discharge thereof, or the exclusion of some washer machines, for example for cleaning and/or maintenance interventions, without compromising the functioning of the washing apparatus 10.

It is clear that modifications and/or additions of parts may be made to the washing apparatus 10 as described heretofore, without departing from the field and scope of the present invention.

For example, the washing apparatus 10 can have the legs 70 of the trolley 21 provided with a spring-type shock-absorber 69, or a piston driven fluid-dynamically, with a leaf spring or a combination of these, such as for example a gas spring, to deaden and eliminate vibrations, oscillations and deviations from the linear path of the trolley 21 on the support plane 23 (fig. 10).

Another advantageous modification to the present invention is to make sliding guides 71 disposed in each housing zone 33 (fig. 9), substantially perpendicular to the axis of alignment T and on which each of the washer machines 11, 12, 13, 14, 15 slidingly rests. In this way it is possible to selectively extract one or more of the washer machines 11, 12, 13, 14, 15 from the housing zones 33 defined by the frame 32, and to insert them therein, for example for possible needs of maintenance and/or replacement.

## Claims

1. Washing apparatus, in particular for heat-disinfection, comprising a plurality of washer machines (11, 12, 13, 14, 15), which are positioned aligned along an axis of alignment (T), and a movement device (19) able to move at least an object-bearing container (20) parallel to said axis of alignment (T), wherein said movement device (19) is provided with:
- a trolley (21) able to transport said container (20) and provided with sliding means (22a, 22b) guided by a guide element (35) which extends parallel to said axis of alignment (T);
- movement means (24) able to move said trolley (20) parallel to said guide element (35);
- a frame (32), disposed along said axis of alignment (T) **characterized in that** said frame (32)comprises at least a first pillar (132) disposed at a first lateral end of said plurality of washer machines (11, 12, 13, 14, 15), a second pillar (232) disposed at a second lateral end of said plurality of washer machines (11, 12, 13, 14, 15) and a plurality of intermediate pillars (332) disposed distanced from each other, according to the sizes of the washer machines (11, 12, 13, 14, 15), and interspersed with the latter so as to define a plurality of housing zones (33) in which said washer machines (11, 12, 13, 14, 15) are able to be positioned, and **in that** said frame (32) further comprises a horizontal bar (31) extending parallel to said axis of alignment (T) and able to support and position, at least partly, said movement means (24).

2. Apparatus as in claim 1, **characterized in that** each of said housing zones (33) is able to house one of said washer machines (11, 12, 13, 14, 15).

3. Apparatus as in claim 1 or 2, wherein each of said washer machines (11, 12, 13, 14, 15) is provided with an internal chamber (41) which is accessed by means of a corresponding aperture (38), **characterized in that** said movement device (19) is positioned below a lower end (43) of each aperture (38).

4. Apparatus as in any claim hereinbefore, **characterized in that** said sliding means (22a, 22b) slides on support means autonomous with respect to said washer machines (11, 12, 13, 14, 15).

5. Apparatus as in claim 4, **characterized in that** said guide element (35) is associated with said support means.

6. Apparatus as in any claim hereinbefore, **characterized in that** said guide element (35) is associated with said frame (32).

7. Apparatus as in claim 5, **characterized in that** said guide element (35) is disposed on plates (36) able to attach said frame (32) to said support means (23).

8. Apparatus as in claim 6, **characterized in that** said horizontal bar (31) supports and positions, directly or indirectly, said guide element (35).

9. Apparatus as in any claim hereinbefore, **characterized in that** said sliding means (22a, 22b) cooperates directly with said guide element (35).

10. Apparatus as in claim 4, **characterized in that** said sliding means (22a, 22b) cooperates directly with said support means.

11. Apparatus as in claim 4, **characterized in that** said support means is a horizontal plane.

12. Apparatus as in claim 11, **characterized in that** said horizontal plane also functions as a base plane for said washer machines (11, 12, 13, 14, 15).

13. Apparatus as in any claim hereinbefore, **characterized in that** said axis of alignment (T) is rectilinear.

14. Apparatus as in any claim from 1 to 12, **characterized in that** said axis of alignment (T) is curvilinear.

15. Apparatus as in any claim hereinbefore, **characterized in that** said trolley (21) comprises shock-absorber means (69).

16. Apparatus as in any claim hereinbefore, **characterized in that** said movement device (19) comprises a second trolley (121) able to translate along the exit side (74) of said washer machines (11, 12, 13, 14, 15).

17. Apparatus as in any claim hereinbefore, **characterized in that** said movement device (19) comprises first mechanical transmission means (29) able to move said trolley (21) and driven by a first motor (25).

18. Apparatus as in any claim hereinbefore, **characterized in that** said movement device (19) comprises a first movement device (45) to move said container (20) onto said trolley (21) driven by a second motor (50) and by second mechanical transmission means (53) and a second movement device (37) able to move said container (20) into one of said washer machines (11, 12, 13, 14, 15) and driven by a third motor (58) and by third mechanical transmission means (61).

19. Apparatus as in claim 17 and 18, **characterized in that** said first motor (25), said second motor (50) and said third motor (58) are compacted and disposed in a single drive unit (62) disposed completely outside said trolley (21).

## Patentansprüche

1. Waschvorrichtung, insbesondere zur Wärmedesinfektion, aufweisend eine Mehrzahl von Waschermaschinen (11, 12, 13, 14, 15), die entlang einer Ausrichtungsachse (T) fluchtend positioniert sind, und eine Bewegungsvorrichtung (19), die imstande ist, wenigstens einen Objekttragenden Container (20) parallel zu der Ausrichtungsachse (T) zu bewegen, wobei die Bewegungsvorrichtung (19) ausgestattet ist mit:
- einem Förderwagen (21), der imstande ist, den Container (20) zu transportieren, und mit Verschiebemitteln (22a, 22b) ausgestattet ist, welche von einem Führungselement (35) geführt werden, das sich parallel zur Ausrichtungsachse (T) erstreckt;
- Bewegungsmitteln (24), die imstande sind, den Förderwagen (20) parallel zum Führungselement (35) zu bewegen;
- einem Rahmen (32), der entlang der Ausrichtungsachse (T) angeordnet ist,
**dadurch gekennzeichnet, dass** der Rahmen (32) aufweist wenigstens eine erste Stütze (132), die an einem ersten seitlichen Ende der Mehrzahl von Waschermaschinen (11, 12, 13, 14, 15) angeordnet ist, eine zweite Stütze (232), die an einem zweiten seitlichen Ende der Mehrzahl von Waschermaschinen (11, 12, 13, 14, 15) angeordnet ist, und eine Mehrzahl von Zwischenstützen (332), die gemäß den Größen der Waschermaschinen (11, 12, 13, 14, 15) im Abstand zueinander angeordnet sind und zwischen denen Letztere eingefügt sind, um eine Mehrzahl von Aufnahmezonen (33) zu definieren, in welchen die Waschermaschinen (11, 12, 13, 14, 15) positioniert werden können, und dass, der Rahmen (32) ferner aufweist eine Horizontalstange (31), die sich parallel zur Ausrichtungsachse (T) erstreckt und imstande ist, die Bewegungsmittel (24) wenigstens teilweise zu stützen und zu positionieren.

2. Vorrichtung wie in Anspruch 1, **dadurch gekennzeichnet, dass** eine jede der Aufnahmezonen (33) imstande ist, eine der Waschermaschinen (11, 12, 13, 14, 15) aufzunehmen.

3. Vorrichtung wie in Anspruch 1 oder 2, wobei eine jede der Waschermaschinen (11, 12, 13, 14, 15) mit einer inneren Kammer (41) ausgestattet ist, die mittels einer korrespondierenden Öffnung (38) zugänglich ist, **dadurch gekennzeichnet, dass** die Bewegungsvorrichtung (19) unter einem unteren Ende (43) einer jeden Öffnung (38) positioniert ist.

4. Vorrichtung wie in einem beliebigen der vorhergehenden Ansprüche, **dadurch gekennzeichnet, dass** sich die Verschiebemittel (22a, 22b) auf einem Stützmittel unabhängig in Bezug auf die Waschermaschienen (11, 12, 13, 14, 15) verschieben.

5. Vorrichtung wie in Anspruch 4, **dadurch gekennzeichnet, dass** das Führungselement (35) dem Stützmittel zugehörig ist.

6. Vorrichtung wie in einem beliebigen der vorhergehenden Ansprüche, **dadurch gekennzeichnet, dass** das Führungselement (35) dem Rahmen (32) zugehörig ist.

7. Vorrichtung wie in Anspruch 5, **dadurch gekennzeichnet, dass** das Führungselement (35) auf Platten (36) angeordnet ist, die imstande sind, den Rahmen (32) an dem Stützmittel (23) zu befestigen.

8. Vorrichtung wie in Anspruch 6, **dadurch gekennzeichnet, dass** die Horizontalstange (31) direkt oder indirekt das Führungselement (35) stützt und positioniert.

9. Vorrichtung wie in einem beliebigen der vorhergehenden Ansprüche, **dadurch gekennzeichnet, dass** die Verschiebemittel (22a, 22b) direkt mit dem Führungselement (35) zusammenwirken.

10. Vorrichtung wie in Anspruch 4, **dadurch gekennzeichnet, dass** die Verschiebemittel (22a, 22b) direkt mit dem Stützmittel zusammenwirken.

11. Vorrichtung wie in Anspruch 4, **dadurch gekennzeichnet, dass** das Stützmittel eine horizontale Ebene ist.

12. Vorrichtung wie in Anspruch 11, **dadurch gekennzeichnet, dass** die horizontale Ebene auch als eine Grundebene für die Waschermaschinen (11, 12, 13, 14, 15) fungiert.

13. Vorrichtung wie in einem beliebigen der vorhergehenden Ansprüche, **dadurch gekennzeichnet, dass** die Ausrichtungsachse (T) geradlinig ist.

14. Vorrichtung wie in einem beliebigen der Ansprüche 1 bis 12, **dadurch gekennzeichnet, dass** die Ausrichtungsachse (T) kurvenförmig ist.

15. Vorrichtung wie in einem beliebigen der vorhergehenden Ansprüche, **dadurch gekennzeichnet, dass** der Förderwagen (21) Stoßdämpfermittel (69) aufweist.

16. Vorrichtung wie in einem beliebigen der vorhergehenden Ansprüche, **dadurch gekennzeichnet, dass** die Bewegungsvorrichtung (19) einen zweiten Förderwagen (121) aufweist, der imstande ist, sich entlang der Ausgangsseite (74) der Waschermaschinen (11, 12, 13, 14, 15) zu verschieben.

17. Vorrichtung wie in einem beliebigen der vorhergehenden Ansprüche, **dadurch gekennzeichnet, dass** die Bewegungsvorrichtung (19) ein erstes mechanisches Übertragungsmittel (29) aufweist, das imstande ist, den Förderwagen (21) zu bewegen, und von einem ersten E-Motor (25) angetrieben wird.

18. Vorrichtung wie in einem beliebigen der vorhergehenden Ansprüche, **dadurch gekennzeichnet, dass** die Bewegungsvorrichtung (19) eine von einem zweiten E-Motor (50) und von zweiten mechanischen Übertragungsmitteln (53) angetriebene, erste Bewegungsvorrichtung (45), um den Container (20) auf den Förderwagen (21) zu bewegen, und eine zweite Bewegungsvorrichtung (37) aufweist, die imstande ist, den Container (20) in eine der Waschermaschinen (11, 12, 13, 14, 15) zu bewegen, und von einem dritten E-Motor (58) und von dritten mechanischen Übertragungsmitteln (61) angetrieben wird.

19. Vorrichtung wie in Anspruch 17 und 18, **dadurch gekennzeichnet, dass** der erste E-Motor (25), der zweite E-Motor (50) und der dritte E-Motor (58) kompaktiert sind und in einer einzelnen Antriebseinheit (62) angeordnet sind, die vollständig außerhalb des Förderwagens (21) angeordnet ist.

## Revendications

1. Appareil de lavage, en particulier de désinfection thermique comprenant une pluralité de machines de lavage (11, 12, 13, 14, 15), qui sont positionnées alignées le long d'un axe d'alignement (T), et un dispositif de déplacement (19) capable de déplacer au moins un conteneur de support d'objet (20) parallèlement audit axe d'alignement (T), dans lequel ledit dispositif de déplacement (19) est doté de :
- un chariot (21) capable de transporter ledit conteneur (20) et doté de moyens de coulissement (22a, 22b) guidés par un élément de guidage (35) qui s'étend parallèlement audit axe d'alignement (T) ;
- des moyens de déplacement (24) capables de déplacer ledit chariot (20) parallèlement audit élément de guidage (35) ;
- un châssis (32) disposé le long dudit axe d'alignement (T) **caractérisé en ce que** ledit châssis (32) comprend au moins un premier pilier (132) disposé au niveau d'une première extrémité latérale de ladite pluralité de machines de lavage (11, 12, 13, 14, 15), un second pilier (232) disposé au niveau d'une seconde extrémité latérale de ladite pluralité de machines de lavage (11, 12, 13, 14, 15) et une pluralité de piliers intermédiaires (332) disposés à distance les uns des autres, selon les tailles des machines de lavage (11, 12, 13, 14, 15) et parsemés parmi ces dernières afin de définir une pluralité de zones de logement (33) dans lesquelles lesdites machines de lavage (11, 12, 13, 14, 15) peuvent être positionnées, et **en ce que** ledit châssis (32) comprend en outre une barre horizontale (31) s'étendant parallèlement à l'axe d'alignement (T) et pouvant supporter et positionner, au moins partiellement lesdits moyens de déplacement (24).

2. Appareil selon la revendication 1, **caractérisé en ce que** chacune desdites zones de logement (33) peut loger au moins l'une desdites machines de lavage (11, 12, 13, 14, 15).

3. Appareil selon la revendication 1 ou 2, dans lequel chacune desdites machines de lavage (11, 12, 13, 14, 15) est prévue avec une chambre interne (41) à laquelle on a accès au moyen d'une ouverture (38) correspondante, **caractérisé en ce que** ledit dispositif de déplacement (19) est positionné au-dessous d'une extrémité inférieure (43) de chaque ouverture (38).

4. Appareil selon l'une quelconque des revendications précédentes, **caractérisé en ce que** lesdits moyens de coulissement (22a, 22b) coulissent sur des moyens de support autonomes par rapport auxdites machines de lavage (11, 12, 13, 14, 15).

5. Appareil selon la revendication 4, **caractérisé en ce que** ledit élément de guidage (35) est associé avec lesdits moyens de support.

6. Appareil selon l'une quelconque des revendications précédentes, **caractérisé en ce que** ledit élément de guidage (35) est associé avec ledit châssis (32).

7. Appareil selon la revendication 5, **caractérisé en ce que** ledit élément de guidage (35) est disposé sur des plaques (36) pouvant fixer ledit châssis (32) auxdits moyens de support (23).

8. Appareil selon la revendication 6, **caractérisé en ce que** ladite barre horizontale (31) supporte et positionne, directement ou indirectement, ledit élément de guidage (35).

9. Appareil selon l'une quelconque des revendications précédentes, **caractérisé en ce que** lesdits moyens de coulissement (22a, 22b) coopèrent directement avec ledit élément de guidage (35).

10. Appareil selon la revendication 4, **caractérisé en ce que** lesdits moyens de coulissement (22a, 22b) coopèrent directement avec ledit moyen de support.

11. Appareil selon la revendication 4, **caractérisé en ce que** lesdits moyens de support sont un plan horizontal.

12. Appareil selon la revendication 11, **caractérisé en ce que** ledit plan horizontal fonctionne également comme un plan de base pour lesdites machines de lavage (11, 12, 13, 14, 15).

13. Appareil selon l'une quelconque des revendications précédentes, **caractérisé en ce que** ledit axe d'alignement (T) est rectiligne.

14. Appareil selon l'une quelconque des revendications 1 à 12, **caractérisé en ce que** ledit axe d'alignement (T) est curviligne.

15. Appareil selon l'une quelconque des revendications précédentes, **caractérisé en ce que** ledit chariot (21) comprend des moyens d'amortissement (69).

16. Appareil selon l'une quelconque des revendications précédentes, **caractérisé en ce que** ledit dispositif de déplacement (19) comprend un second chariot (121) pouvant effectuer un mouvement de translation le long du côté de sortie (74) desdites machines de lavage (11, 12, 13, 14, 15).

17. Appareil selon l'une quelconque des revendications précédentes, **caractérisé en ce que** ledit dispositif de déplacement (19) comprend des premiers moyens de transmission mécanique (29) pouvant déplacer ledit chariot (21) et entraînés par un premier moteur (25).

18. Appareil selon l'une quelconque des revendications précédentes, **caractérisé en ce que** ledit dispositif de déplacement (19) comprend un premier dispositif de déplacement (45) pour déplacer ledit conteneur (20) sur ledit chariot (21) entraîné par un deuxième moteur (50) et par des deuxièmes moyens de transmission mécanique (53) et un second dispositif de déplacement (37) pouvant déplacer ledit conteneur (20) dans l'une desdites machines de lavage (11, 12, 13, 14, 15) et entraîné par un troisième moteur (58) et par des troisièmes moyens de transmission mécanique (61).

19. Appareil selon les revendications 17 et 18, **caractérisé en ce que** ledit premier moteur (25), ledit deuxième moteur (50) et ledit troisième moteur (58) sont compactés et disposés dans une seule unité d'entraînement (62) disposée complètement à l'extérieur dudit chariot (21).
